# EUROPEAN PATENT APPLICATION

(11) **EP 4 756 836 A2**
(43) Date of publication of application: **10.06.2026**
(21) Application number: 26161428.3
(22) Date of filing: 23.06.2021
(51) Int. Cl.: G16H 50/20

(54) **DRUG DELIVERY DEVICE RECOGNITION**

(30) Priority: 25.06.2020 EP 20315322
(62) Divisional of application: 21734007.4
(71) Applicant: SANOFI, 75008 Paris (FR)
(72) Inventor: HELMER, Michael, 65926 Frankfurt am Main (DE); VITT, Martin, 65926 Frankfurt am Main (DE)
(74) Representative: McDougall, James

(57) **Abstract**

A method of detecting a dose of a medicament administered using a drug delivery device is provided. The drug delivery device comprises a dosage dial and a dialled dose indication notch, the dosage dial comprising a plurality of notches. The method comprises: dialling a dose; determining, after dialling a dose, a distance between the dosage dial and a body of the drug delivery device; detecting the relative position of the dialled dose indication notch and the notches provided on the dosage dial; determining the dialled dose based on the detected distance and the relative position of the dialled dose indication notch and the notches provided on the dosage dial.

## Description

### Field

The present disclosure relates to a drug delivery device recognition and devices and systems used in drug delivery device recognition.

### Background

Pen-type drug delivery devices have application where regular injection by users (patients) themselves occurs. This is increasingly common among patients having diabetes where self-treatment enables such patients to conduct effective management of their diabetes.

A drug delivery device may be for example a pre-filled disposable insulin pen. Alternatively, a re-usable pen may be used. A re-usable pen allows replacement of an empty drug cartridge by a new one. Either pen may come with a set of needles that are replaced before each use. The insulin dose to be injected can then for instance be manually selected at the insulin pen by turning a dosage dial and observing the actual dose from a dosage window of the insulin pen. The dose is then injected by inserting the needle into a suitable skin portion and pressing an injection button of the insulin pen.

It may be desirable to provide a user with additional or continued assistance in use of the drug delivery device. For example, the user may need to use more than one drug delivery device, and may struggle to recognize or remember when to use which device.

It may also be advantageous to monitor the status of the device and/or the correct use of the device by the user, either permanently or temporarily (e.g. when the user is still not familiar with the device).

In certain situations, it may be advantageous to verify the device and confirm that the device is authentic (i.e. not e.g. a counterfeiting device).

### Summary

In a first aspect, a system comprising a user device and a drug delivery device is provided, the user device comprising a processor, a memory, a display and a camera; the drug delivery device comprising a body, an injection mechanism and a cap; wherein the memory of the user device stores instructions which, when executed by the processor, cause the user device to: capture, using the camera of the device, at least one image and/or video of a drug delivery device; identify, in the at least one captured image and/or video of the drug delivery device, at least one distinctive feature; compare the at least one distinctive feature identified based on the at least one image and/or video to at least one predefined set of distinctive features, the predefined set of distinctive features being stored in the memory of the user device; identify the drug delivery device based on a match of the at least one distinctive feature identified based on the at least one image and/or video with at least one distinctive features of the predefined set of distinctive features; and output the result of identification of the drug delivery device on the display.

In different embodiments of the first aspect, the system may comprise one or more of the following features:
- the drug delivery device further comprises at least one of the following: a dosage dial for selecting a dose, a dosage window for showing the selected dose, a needle, a cap covering the needle, and a cap covering at least a part of the body;
- in case of no match of the at least one distinctive feature identified based on the at least one image and/or video with at least one distinctive features of the predefined set of distinctive features, the memory of the user device stores instructions which, when executed by the processor, cause the user device to: capture at least one second image and/or video; and identify the drug delivery device based on a match of the at least one distinctive feature identified based on the at least one second image and/or video with at least one distinctive features of the predefined set of distinctive features;
- the memory of the user device stores instructions which, when executed by the processor, further cause the user device to: identify absence of a distinguishing feature; indicate, using the display of the user device, based on the absence of the distinctive feature, that the drug delivery device is likely to be damaged;
- a set of distinctive features comprises one or more of the following: an outer shape of the body of the drug delivery device; a shape of a latch provided on the cap covering at least a part of the body; colour of the of the body of the drug delivery device; dimensions of the body of the drug delivery device;
- a set of distinctive features comprises at least three dots or pixels having a colour different from the rest of the body of the drug delivery device;
- the memory of the user device stores instructions which, when executed by the processor, further cause the user device to: detect the dots or pixels; determine, based on the detection of the dots or pixels, the distance between the drug delivery device and the user device and/or the orientation of the drug delivery device with respect to the user device;
- the drug delivery device comprises a dosage dial, the dosage dial comprises a collar, the body of the drug delivery device comprises a collar, and the collars are positioned facing each other;
- the outermost diameter of the collars is larger than the outermost diameter of the body of the drug delivery device, so that the collars protrude from the body and the dosage dial, respectively, forming ring-like structures.

In a second aspect, a user device comprising a processor, a memory, a display and a camera is provided, wherein the memory of the user device stores instructions which, when executed by the processor, cause the user device to: capture, using the camera of the device, at least one image and/or video of a drug delivery device; identify, in the at least one image and/or video of the drug delivery device, at least one distinctive feature; compare the at least one distinctive feature identified based on the at least one image and/or video to at least one predefined set of distinctive features, the predefined set of distinctive features being stored in the memory of the user device; identify the drug delivery device based on a match of the at least one distinctive feature identified based on the at least one image and/or video with at least one distinctive features of the predefined set of distinctive features.

In a third aspect, a drug delivery device for use in any one of the embodiments described above is provided.

In a fourth aspect, a method of identification of a drug delivery device is provided, comprising: capturing, using the camera of the device, at least one image and/or video of a drug delivery device; identifying, in the at least one image and/or video of the drug delivery device, at least one distinctive feature; comparing the at least one distinctive feature identified based on the at least one image and/or video to at least one predefined set of distinctive features, the predefined set of distinctive features being stored in the memory of the user device; identifying the drug delivery device based on a match of the at least one distinctive feature identified based on the at least one image and/or video with at least one distinctive features of the predefined set of distinctive features.

In a fifth aspect, a method of detecting a dose of a medicament administered using a drug delivery device is provided, the drug delivery device comprising a drug container, the drug container comprising a bung, the method comprising: capturing a first picture of the drug container, wherein the first picture corresponds to a state of the container before administering the dose of the medicament; determining a first position of the bung in the drug container, wherein the first position corresponds to a position of the bung before administering the dose of the medicament; capturing a second picture of the drug container, wherein the second picture corresponds to a state of the container after administering the dose of the medicament; determining a second position of the bung in the drug container, wherein the second position corresponds to a position of the bung after administering the dose of the medicament; calculating, using the first position of the bung and the second position of the bung, the administered dose.

In a sixth aspect, a method of detecting a dose of a medicament administered using a drug delivery device is provided, the drug delivery device comprising a dosage dial and a dialled dose indication notch, the dosage dial comprising a plurality of notches, the method comprising: dialling a dose; determining, after dialling a dose, distance L6 between the dosage dial and the body of the drug delivery device; detecting the relative position of the dialled dose indication notch and the notches provided on the dosage dial; determining the dialled dose based on the detected distance L6 and the relative position of the dialled dose indication notch and the notches provided on the dosage dial.

The distinctive features referred to above may generally comprises one or more of the following:
- an outer shape of the body of the drug delivery device;
- a shape of a latch provided on the cap covering at least a part of the body;
- colour of the of the body of the drug delivery device;
- dots or pixels having a colour different from the rest of the body of the drug delivery device;
- data matrix code or QR code;
- a shape and/or colour of the dosage window;
- a shape and/or colour of the dosage dial;
- a shape and/or colour of the injection button;
- a shape and/or colour of a drug window;
- a distance between the dosage dial and the body of the drug delivery device ;
- closure features on the cap covering at least a part of the body and the corresponding portion of the body;
- notches or protrusions provided on the cap covering at least a part of the body, the body, or both the cap and the body;
- a number and position of notches on the dosage dial;
- a length of the drug delivery device;
- a diameter of the body of the drug delivery device;
- a diameter of the injection button;
- a diameter of the dosage dial.

### Brief Description of the Figures

Specific embodiments of the present invention will now be described, by way of example only, with reference to the accompanying drawings in which:
Fig 1 is a schematic view of the system of a drug delivery device and a user device;
Fig 2 is an exploded view of a drug delivery device;
Fig 3 is a view of example distinctive features of the drug delivery device;
Fig 4 is a view of example distinctive features of the drug delivery device;
Fig 5 is a detail of the injection button part of the drug delivery device;
Fig 6 is a detail of a cap attachment to a body of the drug delivery device;
Fig 7 is an upper view of a dosage dial provided on the drug delivery device;
Fig 8 is a schematic view of a user device;
Fig 9 is a flow chart showing a method of recognizing a device;
Fig 10 is a flow chart showing a method of recognizing administered dose;
Fig 11a and 11b show an example of a drug container with a bung;
Fig 12 shows an example of a container with a bung as seen through a drug window of a drug delivery device.

### Detailed Description

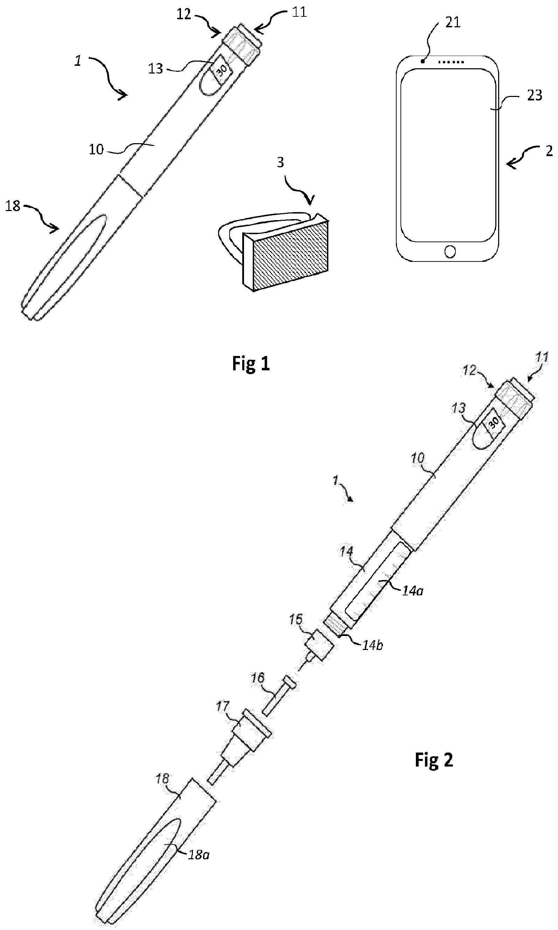

An exemplary system according to the present invention, as seen in Fig 1, comprises a drug delivery device 1 and a user device 2, 3. The drug delivery device 1 may be e.g. an insulin pen. The drug delivery device may be any other pen-type injection device. The user device 2, 3 may be e.g. a mobile phone 2, a tablet, or a PC. The user device in the form of a mobile phone 2 has a camera 21 and a display 23 associated with the user device 2. In an embodiment, the user device 2, 3 may be a device for augmented reality or virtual reality 3. The device for augmented reality (AR) or virtual reality (VR) may be provided in the form of AR / VR glasses 3. A camera and a display may be associated with the AR / VR glasses 3. In an embodiment, the user device 2, 3 may be a mobile phone 2 which cooperates with a device for augmented reality or virtual reality 3.

Fig 2 is an exploded view of an example drug delivery device 1. An example of such device is Sanofi's Solostar (TM) insulin injection pen. The drug delivery device 1 may be any suitable pen-type or auto-injector-type drug delivery device.

The drug delivery device 1 comprises a housing 10. In general, the housing 10 is provided with an injection mechanism, e.g. an injection button 11, a dosage dial 12, a dosage window 13, and a container region 14. A needle 15 can be affixed to the container region 14. The housing is further provided with a cap 18, which covers the container region 14 when the device 1 is not in use (and the needle 15 is not attached). Before the needle 15 is affixed to the housing 1, the needle 15 is protected by an inner needle cap 16 and an outer needle cap 17.

The housing 10 is provided with a container region 14 to which the needle 15 can be affixed. The container region 14 contains a container. The container contains the drug to be injected. The drug may be e.g. insulin. The container may be e.g. an insulin container.

The container region 14 may comprise a drug window 14a. The drug window 14a is for example an opening covered in glass, clear plastic or other transparent material through which the user can see the container and/or the contents of the container).

An appropriate dose of a drug can be selected by turning the dosage dial 12. Turning the dosage dial 12 may cause the dosage dial 12 to extend helically away from the body 10 of the drug delivery device 1 along the longitudinal axis of the body 10. The extension may correspond to the dose dialled (described below).

Turning the dosage dial 12 may cause a mechanical clicker to provide haptic and acoustic feedback to a user. The numbers displayed in dosage window 13 are present on a sleeve by printing and the sleeve is contained in housing 10 and mechanically interacts with a piston in the insulin container, which is positioned in the container region 14. When needle 15 is stuck into a skin portion of a patient and then injection button 11 is pushed, the insulin dose displayed in dosage window 13 will be ejected from the injection device 1. When the needle 15 of the injection device 1 remains for a certain time in the skin portion after the injection button 11 is pushed, a high percentage of the dose is injected into the patient's body. Ejection of the insulin dose also causes a mechanical click sound, which is different from the sounds produced when using dosage dial 12.

The selected dose is displayed via dosage window 13, for instance an insulin dose in multiples of so-called International Units (IU), wherein one IU is the biological equivalent of about 45.5 micrograms of pure crystalline insulin (1/22 mg). An example of a selected dose displayed in dosage window 13 may, for instance, be 30 IU, as shown in Figs 1-5.

The user device 2, 3 may contain suitable software (e.g. a suitable application) which assists the user in handling the drug delivery device 1. For example, the user device 2, 3 may contain suitable software which is able to recognize a particular drug delivery device 1 and then, based on the recognition of the drug delivery device 1, provide the user with assistance. Recognition of the drug delivery device 1 is described below.

For example, the user device 2, 3 may be able to provide or enable one or more of the following functions: indicate to the user whether the user has selected a correct drug delivery device 1; advise the user on the status of the drug delivery device 1 (e.g. advise the user in case a component of the drug delivery device 1 is not functional, or not fully functional); advise the user on the status of the drug loaded in the drug delivery device 1 (e.g. in case the drug is or appears to be past its expiration date); supervise the user in their use of the drug delivery device 1; guide the user through the use of the drug delivery device 1; indicate whether the drug delivery device 1 is authentic (i.e. not a counterfeiting device); verify the dose administered by the drug delivery device 1; etc.

In use, the user positions the user device 2, 3 and the drug delivery device 1 such that the camera 21 of the user device 2, 3 is able to capture an image, a video, or both an image and a video of the drug delivery device 1. The image or video may be captured by a camera 21 positioned on the front of the user device 2, 3 (i.e. on the same side of the user device 2, 3 as the display 23). Alternatively or in addition, the image or video may be captured by another camera, such as a camera (not shown) positioned on the back of a mobile phone 2 or tablet, an external webcam (not shown) associated with a desktop or laptop computer, a camera (not shown) associated with the AR/VR glasses 3 etc.

The drug delivery device 1 has one or more distinctive features 101-112, L1-L6, D1-D3. The user device 2, 3 stores suitable software enabling the user device 2, 3 to identify the distinctive features 101-112, L1-L6, D1-D3 and compare them to a pre-determined set of distinctive features associated with a particular device. The user device 2, 3 therefore enables recognition of the drug delivery device 1. The distinctive features 101-112, L1-L6, D1-D3 may enable the user device 2, 3 to identify the status of the drug delivery device 1.

Preferably, the user device 2, 3 stores more than one distinctive feature 101-112, L1-L6, D1-D3 of a given drug delivery device 1. If the user device 2, 3 stores more than one distinctive feature 101-112, L1-L6, D1-D3 of a given drug delivery device 1, the drug delivery device 1 may be identified by the user device 2, 3 with more accuracy. If the user device 2, 3 stores more than one distinctive feature 101-112, L1-L6, D1-D3 of a given drug delivery device 1, it may be possible to recognize one or more of the following: position of the drug delivery device 1, orientation of the drug delivery device 1 with respect to the user device 2, 3, distance of the drug delivery device 1 from the user device 2, 3, etc. Alternatively or in addition, if the user uses the user device 2, 3 for capturing a video rather than a static picture, it may be possible to identify the movements of the drug delivery device 1, changes in its position, orientation, distance from the user device 2, 3 etc.

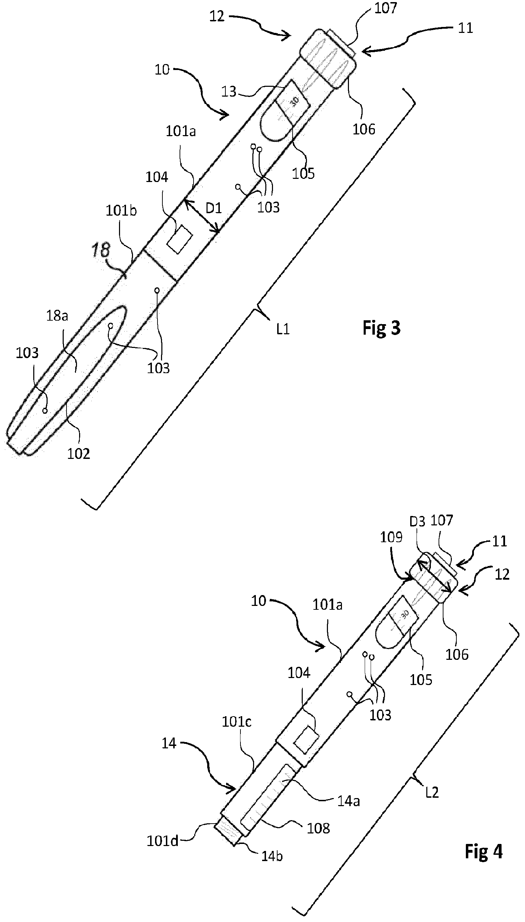

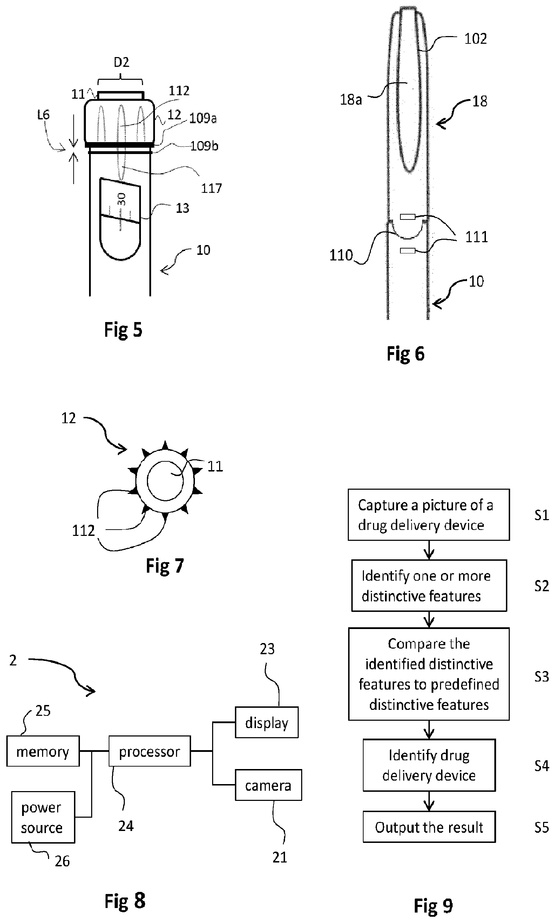

Example features which may serve as distinctive features 101-112, L1-L6, D1-D3 are shown in Figs 3-7. The distinctive features 101-112, L1-L6, D1-D3 may be for example:
- outer shape 101a, 101b of the body 10 of the drug delivery device 1 and the cap 18 which is in place over the container region 14;
- outer shape 101a, 101c, 101d of the body 10 of the drug delivery device 1 and the container region 14 (i.e. with the cap 18 removed);
- outer shape of the body 10 of the drug delivery device 1, the container region 14 and the needle 15;
- shape 102 of a latch 18a (the latch 18a being provided on the cap 18);
- colour of the of the body 10 of the drug delivery device 1;
- dots or pixels 103 having a colour different from the rest of the body 10 of the drug delivery device 1;
- a code 104 such as data matrix code or QR code provided on the body 10, on the cap 18, or both on the body 10 and the cap 18;
- the shape and/or colour 105 of the dosage window 13;
- the shape and/or colour 106 of the dosage dial 12;
- the shape and/or colour 107 of the injection button 11;
- the shape and/or colour 108 of the drug window 14a;
- the distance L6 between the dosage dial 12 and the body 10 of the drug delivery device 1;
- closure features 110 on the cap 18 and the corresponding portion of the body 10 (see Fig 6);
- notches or protrusions 111 provided on the cap 18, the body 10, or both the cap 18 and the body 10 (see Fig 6);
- number and position of notches 112 on the dosage dial 12 (see Fig 7);
- the length L1 of the drug delivery device 1 including the cap 18 (see Fig 3);
- the length L2 of the drug delivery device 1 without the cap 18 and without the needle 15 (see Fig 4);
- the length L3 of the drug delivery device 1 without the cap 18 and without the inner and outer needle caps 16-17 but with the needle 15 attached (not shown);
- the length L4, L5 of the drug delivery device 1 without the cap 18 but with one or both of the inner and outer needle caps 16-attached (not shown);
- the diameter D1 of the body 10 of the drug delivery device 1 (see Fig 3);
- the diameter D2 of the injection button 11;
- the diameter D3 of the dosage dial 12 (see Fig 4);
- the ratio of any two of the lengths L1-L6 and/or diameters D1-D3 (in general, any of the ratios Lx/Ly, Dx/Dy, or Lx/Dy, where x, y stand for the number of the respective length or diameter);
- distances (not shown) between any two of the distinctive features 101-112 listed above and/or ratios of any two of these distances;
- one or more NFC tags, e.g. a RFID tag, provided in the cap 18, the body 10 or both the cap 18 and the body 10 of the drug delivery device 1 (not shown).

The distinctive features 101-112, L1-L6, D1-D3 may be used alone or in combination. The user device 2, 3 may recognize one or more of the features in a given drug delivery device 1. Any one of the features 101-112, L1-L6, D1-D3 listed above can be used for drug delivery device recognition in combination with any other of the features 101-112, L1-L6, D1-D3. The user device 2, 3 may store any subset of the above listed distinctive features 101-112, L1-L6, D1-D3, including all the distinctive features 101-112, L1-L6, D1-D3. Recognition by the user device 2, 3 of any of the distinctive features 101-112, L1-L6, D1-D3 listed above can be combined with recognition by the user device 2, 3 of any other of the distinctive features 101-112, L1-L6, D1-D3. The drug delivery device 1 may be provided with any subset of the above listed distinctive features 101-112, L1-L6, D1-D3, including **all** the distinctive features 101-112, L1-L6, D1-D3. A drug delivery device 1 may be provided with any one of the features 101-112, L1-L6, D1-D3 listed above in combination with any other of the features 101-112, L1-L6, D1-D3.

The user device 2, 3 stores predefined distinctive features of a given drug delivery device 1 (such as any one of the distinctive features 101-112, L1-L6, D1-D3, a subset of the distinctive features 101-112, L1-L6, D1-D3 listed above, or all of the distinctive features 101-112, L1-L6, D1-D3 listed above). The user device 2, 3 is capable of comparing the distinctive features measured from the image or video captured by the camera 21 to the predefined distinctive features stored in the memory 25 of the user device 2, 3 to identify which drug delivery device 1 is presented to the camera 21.

For example, the user may own two different drug delivery devices. Each of these drug delivery devices has a set of distinctive features 101-112, L1-L6, D1-D3. The first set of distinctive features 101-112, L1-L6, D1-D3 of the first drug delivery device differs in at least one of the distinctive features, and preferably in more than one distinctive feature, from the second set of distinctive features 101-112, L1-L6, D1-D3 of the second drug delivery device. The user device 2, 3 stores the first set and the second set of distinctive features 101-112, L1-L6, D1-D3 of the first and second drug delivery device, respectively. When the user presents one of their drug delivery devices to the camera 21 of the user device 2, 3, the user device 2, 3 captures an image of the drug delivery device presented to the camera 21. Based on the distinctive features 101-112, L1-L6, D1-D3, the user device 2, 3 uses the captured image to derive the distinctive features of the drug delivery device 1 shown to the camera 21. The user delivery device 1 compares the distinctive features derived from the image with the stored first and second sets of distinctive features 101-112, L1-L6, D1-D3. The user device 2, 3 then identifies which one of the first and the second set matches the distinctive features derived from the image, and determines which one of the user-owned drug delivery devices has been presented to the camera 21.

In an embodiment, if the distinctive features do not match any one of the user-owned drug delivery devices, the user device 2, 3 may request a second image to be taken.

The user device 2, 3 may request the second image to be taken from a different angle, a different distance, or having the drug delivery device 1 positioned in a different orientation with respect to the camera 21 of the user device 2, 3; any combination of these could be used. A second image taken from a different angle may be advantageous in that a different (e.g. easily detectable) distinctive feature may be presented to the camera 21.

In an embodiment, if the distinctive features do not match any one of the user-owned drug delivery devices, the user device 2, 3 may indicate that the drug delivery device presented to the camera 21 is unknown to the user device 2, 3.

In an embodiment, if the distinctive features of the drug delivery device 1 do not match the predefined set of distinctive features of any one of the user-owned drug delivery devices, the user device 2, 3 may request a number of second images of the drug delivery device 1 to be taken. If, after taking a threshold number of second images, the distinctive features of the drug delivery device 1 are still not identified to match any one of the user-owned drug delivery devices, the user device 2, 3 may indicate that the drug delivery device presented to the camera 21 is unknown to the user device 2, 3.

In an embodiment, the user device 2, 3 may identify the drug delivery device if more than a threshold number of features are recognized as matching that given drug delivery device. For example, if the user device 2, 3 identifies five different distinctive features 101-112, L1-L6, D1-D3, a positive match is indicated.

In an embodiment, the user device 2, 3 may be adapted to recognize that some of the device features corresponding to the distinguishing features 101-112, L1-L6, D1-D3 are missing or are damaged. For example, the dosage dial 12 has diameter D3, collar 109a, notches 112, and corresponding distance L6 between the dosage dial 12 and the body 10 of the drug delivery device 1. Based on absence of one or more of these distinctive features, the user device 2, 3 may indicate that the dosage dial may be missing or damaged. Determination of an absence of a distinctive feature may be based e.g. on failure to identify the distinctive feature in one or more captured images, preferably in more than one subsequent images. For example, if the notches 112 of the dosage dial 12 are impossible to identify but the dosage dial 12 is identified and all other distinctive features 101-112, L1-L6, D1-D3 are identified and correspond to the given drug delivery device 1, the user device 2, 3 may indicate that the dosage dial 12 may be damaged and needs to be checked.

In one embodiment, the user device 2, 3 may capture the image of the drug delivery device 1 before and after use. Based on the differences between the images, the user device 2, 3 may be able to indicate errors in handling the drug delivery device 1.

The recognition of the drug delivery device 1 based on the recognition of the outer shape of the device 101a-d as well as the shapes of various other features (e.g. the cap latch 18a, the dosage window 13, the dosage dial 12, the injection button 11 or the drug window 14a) may depend on the distance of the drug delivery device 1 from the camera 21 of the user device 2, 3. Alternatively or in addition, the recognition may be based on multiple components (e.g. at least two of the following: the body 10, the cap latch 18a, the dosage window 13, the dosage dial 12, the injection button 11 or the drug window 14a) corresponding in shape to a predefined shape of a known drug delivery device 1.

The recognition of the drug delivery device 1 based on the recognition of the dimensions of parts of the drug delivery device 1 (such as the lengths L1 to L6 and the diameters D1 to D3) may depend on the distance of the drug delivery device 1 from the user device 2, 3.

The distance of the drug delivery device 1 from the user device 2, 3 may be determined e.g. based on known ratio of distances and/or relative positions between individual distinctive features 101-112, L1-L6, D1-D3.

The distance of the drug delivery device 1 from the user device 2, 3 may be determined e.g. based on known ratio of distances and/or relative positions between dots or pixels 103 or between groups of dots or pixels 103.

The dots or pixels 103 may be located on the body 10 of the drug delivery device 1 in a predefined pattern. Alternatively or in addition, the dots or pixels 103 may be located on the cap 18 in a predefined pattern. In an embodiment, there are at least three dots or pixels 103 on the drug delivery device 1. In an embodiment, there are at least six dots or pixels 103 on the drug delivery device 1.

The colour of the dots or pixels 103 may be different from the colour of the body 10 of the drug delivery device 1. The colour of the dots or pixels 103 may be such that the dots or pixels 103 are only detectable by the user device 2, 3. The colour of the dots or pixels 103 may be such as to enable colour detection under different light and environmental conditions, for example when the light conditions are adverse. For example, the dots or pixels 103 may provide high contrast against the colour of the body 10 in order to be visible under dim light. The dots or pixels 103 may be of a colour which lights up when a picture is taken by the user device 2, 3 using flash light.

The dots or pixels 103 may be positioned in a pattern which is recognized by the user device 2, 3. The user device 2, 3 may determine the relative positions of the individual dots or pixels 103 as compared to other dots or pixels 103, or relative distances between groups of individual dots or pixels 103 relative to other groups of individual dots or pixels 103. In an embodiment, the user device 2, 3 may determine both the relative positions and relative distances between the individual dots or pixels 103, or between groups of individual dots or pixels 103.

The distance L6 may depend on the dose dialled. In such case, the distance L6 may correspond in a predefined manner to the dose displayed in the dosage window 13. In an embodiment, the dose displayed in the dosage window 13 may be recognized by a suitable software such as OCR software provided in the user device 2, 3. The dose and the distance L6 may then be correlated.

The recognition of the drug delivery device 1 may be based on recognition that the pattern of the dots or pixels 103 provided on the drug delivery device 1 corresponds to a predefined pattern. Alternatively or in addition, the recognition of the drug delivery device 1 may be based on recognizing the relative distances between the dots or pixels 103.

An appropriate dose of a drug can be selected by turning the dosage dial 12. When the dosage dial 12 is turned, the distance L6 between the body 10 and the dosage dial 12 (also called dial extension) increases. The distance L6 between the body 10 and the dosage dial 12 depends on the dose set and displayed in the dosage window 13. Typically, the distance L6 is in order of millimetres, e.g. 0.1 mm to 50 mm.

The distance between the drug delivery device 1 and the user device 2, 3 may be based on relative distances between the individual dots or pixels 103 or groups of individual dots or pixels 103. The user device 2, 3 compares the measured distances between the individual dots or pixels 103 or groups of individual dots or pixels 103 to predefined (known) distances between the individual dots or pixels 103 or groups of individual dots or pixels 103 on a known drug delivery device 1, and, based on the comparison, derive the distance between the drug delivery device 1 and the user device 2, 3.

Due to the typical values of the distance L6, measuring of the distance L6 may be prone to error. This is especially true when no dose or low dose is selected and/or when the light conditions are adverse. In such circumstances, the camera 21 of the user device 2, 3 may provide insufficient detail (or insufficient resolution) in the captured image for the user device 2, 3 to be able to determine the distance L6 accurately.

In order to enhance correct measurement of the distance L6 using the camera 21 of the user device 2, 3, the body 10 of the drug delivery device 1 and/or the dosage dial 12 may be provided with one or more contrast features. Contrast features may be features which are clearly visible against the other features, such as features provided in contrast colour, features protruding above the surrounding features, etc.

An example of a contrast feature is a collar 109a (see Fig. 5), provided on the dosage dial 12. The collar 109a faces the body 10 of the drug delivery device 1. Alternatively or in addition, the body 10 of the drug delivery device 1 may be provided with collar 109b (see Fig. 5). The collar 109b is positioned on the end of the body 10 opposite the needle 15. The collar 109b faces the dosage dial 12. The collars 109a, 109b may be provided in a colour different from the colour of the body 10 and the dosage dial 12, respectively. For example, the colour of the collars 109a, 109b may be contrasting with the colour of the body 10 of the drug delivery device 1. The outermost diameter of the collars 109a, 109b may be larger than the outermost diameter of the body 10 of the drug delivery device 1, so that the collars 109a, 109b protrude from the body 10 and/or the dosage dial 12, respectively, forming ring-like structures. For example, the collars 109a, 109b may be raised by 0.5-2 mm above the body 10 of the drug delivery device 1.

Further examples of contrast features are described below. In this context, contrast colour is a colour which is visible towards the surrounding features and/or the drug contained in the container. For example, the body 10 of the drug delivery device 1 may be provided in a dark colour (such as black, grey, dark green or brown), while the contrast features may be provided in a transparent colour (yellow, orange) or in a light colour (such as white, beige, yellow or red). For example, the body 10 of the drug delivery device 1 may be provided in a light colour (such as white, light grey or beige), while the contrast features may be provided in a transparent colour (yellow, orange) or in a dark colour (such as black, dark grey, brown, dark green, dark blue or red).

With at least one collar 109a, 109b as described above, the camera 21 of the user device 2, 3 may be able to use the at least one collar 109a, 109b as a higher-contrast detail, providing the user device 2, 3 with better quality data to determine the distance L6 and allowing the user device 2, 3 to achieve a more accurate determination of the distance L6. In an embodiment, the dosage dial 12 is provided with the collar 109a and the body 10 of the drug delivery device 1 is provided with the collar 109b. The collars 109a, 109b face each other, as shown in Fig 5. Providing two collars 109a, 109b enhances the provision of the higher contrast detail and thus allows more accurate identification of the distance L6.

The cap 18 and the corresponding part of the body 10 of the drug delivery device 1 may be provided with closure features 110. The closure features 110 may be for example a distinct shape along the outer periphery of the cap 18 in the region where the cap 18 engages the body 10 of the drug delivery device 1, and a corresponding distinct shape on the corresponding part of the body 10. Alternatively or in addition, the closure features 110 may be a distinct colour and/or distinct lining along the outer periphery of the cap 18 in the region where the cap 18 engages the body 10 of the drug delivery device 1, and a corresponding distinct colour and/or distinct lining on the corresponding part of the body 10. The recognition of the distinct shape may be enhanced by providing the closure features 110 with a coloured lining or a protrusion.

The cap 18 may be provided with one or more notches or protrusions 111. For easier detection by the user device 2, 3, the notches or protrusions 111 may be emphasized by a different colour to the rest of the cap 18. Same or similar notches or protrusions 111 may be provided on the body 10 of the drug delivery device 1. In an embodiment, there may be one notch or protrusion 111 provided on the cap 18, and a corresponding notch or protrusion 111 provided on the body 10. These notches or protrusions 111 may be aligned along the longitudinal axis of the drug delivery device 1. In some embodiments (not shown), there may be more than two notches or protrusions 111. The notches or protrusions 111 may be arranged in another configuration than along a line (e.g. a triangle, such as an equilateral triangle, a rectangle, or any other suitable shape).

The dosage dial 12 may be provided with a number of notches 112. For example, there may be 10 notches 112, each corresponding to 2 IU of insulin. There may other number of notches 112. Alternatively or in addition to helping in indicating dosage, the notches 112 may serve as grip features. Preferably, the notches 112 protrude from the dosage dial 12. For increased contrast and easier processing of a picture captured by the user device 2, 3, the notches 112 may be provided in a colour different to the dosage dial 12, for example in a contrast colour. This is further described below.

The length L1 of the drug delivery device 1 including the cap 18 may be measured between the tip (i.e. the outermost point) of the closed cap 18 and the end point (i.e. the outermost point) of the injection button 11.

The length L2 of the drug delivery device 1 without the cap 18 and without the needle 15 may be measured between the tip (i.e. the outermost point) of the needle mount 14b and the end point (i.e. the outermost point) of the injection button 11.

The length L3 of the drug delivery device 1 without the cap 18 and without the inner and outer needle caps 16-17 but with the needle 15 attached may be measured between the tip (i.e. the outermost point) of the needle 15 and the end point (i.e. the outermost point) of the injection button 11. Alternatively, the length L3 may be measured between the point from which the needle 15 protrudes and the end point (i.e. the outermost point) of the injection button 11.

The length L4 of the drug delivery device 1 without the cap 18 but with the inner needle cap 16 attached may be measured between the tip (i.e. the outermost point) of the inner needle cap 16 and the end point (i.e. the outermost point) of the injection button 11. Similarly, the length L5 of the drug delivery device 1 without the cap 18 but with the outer needle cap 17 attached may be measured between the tip (i.e. the outermost point) of the outer needle cap 17 and the end point (i.e. the outermost point) of the injection button 11.

The diameter D1 of the body 10 of the drug delivery device 1 may be measured in the widest point of the drug delivery device 1. Alternatively, the diameter D1 may be measured in a pre-defined location, for example along the line of contact between the body 10 and the cap 18, along the line where the dosage dial 12 is positioned, or in any other suitable location. The dots or pixels 103 may be used as a lead to measure the diameter D1 in the correct location.

The diameter D2 of the injection button 11 may be measured at the outer-most region of the injection button 11. Alternatively, the diameter D2 may be measured in the widest point of the injection button 11.

Diameter D3 of the dosage dial 12 may be measured at one of the ends (outer-most regions) of the dosage dial 12. The diameter D3 may be measured along the collar 109a. Alternatively, the diameter D2 may be measured in the widest point of the dosage dial 12.

The user device 2, 3 (shown schematically in Fig 8) comprises a processor 24, a memory 25, a camera 21, a display 23, and a power source 26. The memory 25 stores suitable software (e.g. a software application) for the drug delivery device 1 recognition, as described above. The processor 24 executes the software in accordance with what has been described above.

In use, the user captures an image of a drug delivery device 1, using the camera 21 of the user device 2, 3 (Fig 9, step S1). The captured image is then processed by the user device 2, 3, and one or more distinctive features 101-112, L1-L6, D1-D3 are identified in the captured image (step S2). The identified distinctive features 101-112, L1-L6, D1-D3 are compared with distinctive features stored in the user device 2, 3 (step S3). The drug delivery device is then identified (step S4), and the result is output (step S5).

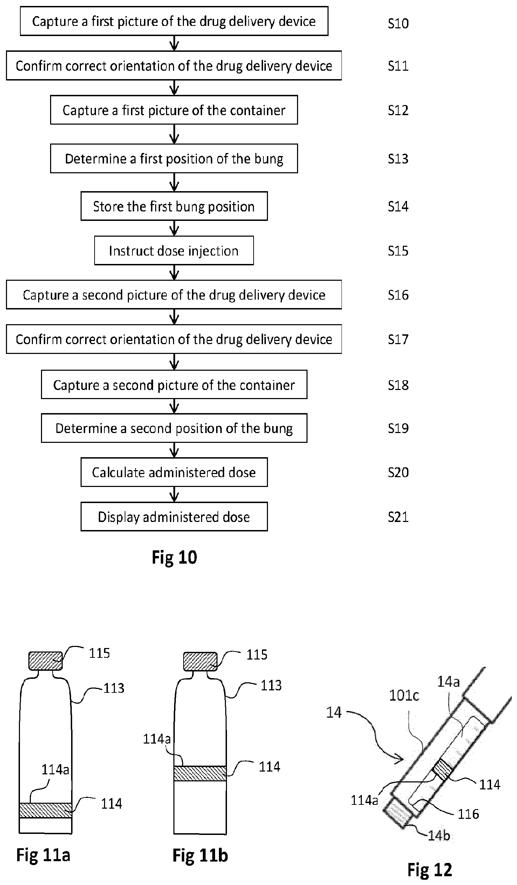

The above-described devices and methods may be employed for example in a method illustrated in Fig 10 (described below).

Figs 11a, b show an example of a container 113 that may be used with the drug delivery device 1. It will be appreciated that the container 113 may be replaced by any suitable alternative. For example, a single-use pen-type drug delivery device may use an integrated container rather than a container shown in Figs 11a, b. Container 113 may be replaceable in the drug delivery device 1.

The container 113 comprises a bung 114 and a closure 115. The closure engages the needle 15 via a needle interface (not shown). The bung 114 closes a first or distal end of the container 113. The closure 115 closes a second or proximal end of the container 113.

The bung 114 is movable within the container 113. Movement of the bung 114 pushes the medicament contained in the container 113 out into the needle 15 and out of the drug delivery device 1. Fig 11a shows an example configuration of the container 113 before a dose is administered. Fig 11b shows an example configuration of the container 113 after a dose is administered. Figs 11a, b show that after the dose is administered, the bung 114 is moved from a first position closer to the distal end of the container 113 into a second position further from the distal end and closer to the proximal end of the container 113.

During use of the drug delivery device 1, the container is contained in the container region 14. The container 113 and the bung 114 may be observed through a drug window 14a. The position of the bung 114 and/or the change of position of the bung 114 may be observed through the drug window 14a.

The user device 2, 3 may be configured to perform a method illustrated in Fig 10. In step S10, the user device 2, 3 captures a first picture of the drug delivery device 1. In step S11, using at least one of the distinctive features 101-112, L1-L6, D1-D3, the user device 2, 3 may determine the position and orientation of the drug delivery device 1.

Optionally, after confirming the position and orientation of the drug delivery device 1, the user device 2, 3 may capture a first picture of the container 113 (step S12).

From the first picture of the drug delivery device 1 and/or from the first picture of the container 113, a first position of the bung 114 may be determined (step S13). The first position of the bung 114 typically corresponds to a position of the bung 114 before a dose is administered.

The user device 2, 3 stores the first bung position (step S14).

The user device 2, 3 may instruct the user to inject the dose (step S15). The user device 2, 3 may optionally require a confirmation from the user that the dose has been injected.

In step S16, the user device 2, 3 captures a second picture of the drug delivery device 1.

In step S17, using at least one of the distinctive features 101-112, L1-L6, D1-D3, the user device 2, 3 may determine the position and orientation of the drug delivery device 1.

Optionally, after confirming the position and orientation of the drug delivery device 1, the user device 2, 3 may capture a second picture of the container 113 (step S18).

From the second picture of the drug delivery device 1 and/or from the second picture of the container 113, a second position of the bung 114 may be determined (step S19).

The second position of the bung 114 typically corresponds to a position of the bung 114 after a dose is administered. From the known (pre-stored) parameters of the cartridge 113 and the known first and second positions of the bung 114, the administered dose may be calculated (step S20).

Optionally, the administered dose may be displayed on the display 23 of the user device 2, 3 (step S21). Alternatively or in addition, information on whether a correct dose has been administered may be displayed. For example, if it is determined that the dose corresponds to the (pre-stored or pre-selected) user's recommended dose, a confirmation that a correct dose has been administered may be displayed.

To aid the bung position recognition, the container 113 and/or the bung 114 and/or the drug window 14a may comprise at least one contrast feature. For example, the inner face of the bung 114a may be provided in a contrast colour and/or with a contrast lining. Similarly, the drug window may have at least one of its boundaries 116 (shown in Fig 12) provided in a contrast colour. Alternatively or in addition, a contrast colour line or slot (not shown) may be provided in or close to the position of the boundary 116. It will be clear to the skilled person that a similar contrast feature may be provided on the container (e.g. in a position close to the proximal end, positioned such that after insertion of the container into the container region, this contrast feature is visible through the drug window 14a). In this context, contrast colour is a colour which is visible towards the other features and/or the drug contained in the container. For example, the container region 14 may be provided in a dark colour (such as black, grey, dark green or brown), the bung 114 may also be provided in a dark colour (same or different from the container region 14 colour), while the contrast features may be provided in a transparent colour (yellow, orange) or in a light colour (such as white, beige, yellow or red).

Alternatively or in addition to measuring the position of the bung 114 as described above, the user device may measure the dial extension, or distance L6. For example, the distance L6 may be measured between the collars 109a, b, as illustrated Fig 5. For example, a dose increase of 1U may increase the distance between the body 10 and the dosage dial 12 by 0.4-0.5 mm, in particular 0.425 mm. For example, dose of 10U may increase the distance from 0mm (0U) to 4-5 mm, in particular 4.25 mm. For example, one full rotation of the dosage dial may be 20U, i.e. 8-10mm, in particular 8.5 mm. Other suitable values of movement by unit may be used.

To enable a more precise measurement of the distance L6, the user device 2, 3 may measure the distance along the longitudinal axis of the drug delivery device 1. To assess the position of the longitudinal axis of the drug delivery device 1, one or more of the distinctive features 101-112, L1-L6, D1-D3 may be used to derive the position and orientation of the drug delivery device 1 with respect to the user device 2, 3.

Using measuring the position of the bung 114 together with measuring the distance L6 helps reduce the influence of axial device design tolerances.

Advantageously, the dosage dial 12 may comprise a notch 112 for each even dose number (each 2U). The user device 2, 3 may measure the alignment of the respective notches 112 with respect to the longitudinal axis of the drug delivery device 1 and/or a dialled dose indication notch 117 (shown in Fig 5). If a notch is aligned with the longitudinal axis and/or the dialled dose indication notch 117 or is within 0.5mm of the longitudinal axis and/or the dialled dose indication notch 117, this serves as an indication that an even dose has been dialled (2U, 4U, etc.). If the longitudinal axis and/or the dialled dose indication notch 117 are positioned between two notches 112, this indicates that an odd dose has been dialled.

The terms "drug" or "medicament" are used synonymously herein and describe a pharmaceutical formulation containing one or more active pharmaceutical ingredients or pharmaceutically acceptable salts or solvates thereof, and optionally a pharmaceutically acceptable carrier. An active pharmaceutical ingredient ("API"), in the broadest terms, is a chemical structure that has a biological effect on humans or animals. In pharmacology, a drug or medicament is used in the treatment, cure, prevention, or diagnosis of disease or used to otherwise enhance physical or mental well-being. A drug or medicament may be used for a limited duration, or on a regular basis for chronic disorders.

As described below, a drug or medicament can include at least one API, or combinations thereof, in various types of formulations, for the treatment of one or more diseases. Examples of API may include small molecules having a molecular weight of 500 Da or less; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more drugs are also contemplated.

The drug or medicament may be contained in a primary package or "drug container" adapted for use with a drug delivery device. The drug container may be, e.g., a cartridge, syringe, reservoir, or other solid or flexible vessel configured to provide a suitable chamber for storage (e.g., short- or long-term storage) of one or more drugs. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of the pharmaceutical formulation to-be-administered (e.g., an API and a diluent, or two different drugs) separately, one in each chamber. In such instances, the two chambers of the dual-chamber cartridge may be configured to allow mixing between the two or more components prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drugs or medicaments contained in the drug delivery devices as described herein can be used for the treatment and/or prophylaxis of many different types of medical disorders. Examples of disorders include, e.g., diabetes mellitus or complications associated with diabetes mellitus such as diabetic retinopathy, thromboembolism disorders such as deep vein or pulmonary thromboembolism. Further examples of disorders are acute coronary syndrome (ACS), angina, myocardial infarction, cancer, macular degeneration, inflammation, hay fever, atherosclerosis and/or rheumatoid arthritis. Examples of APIs and drugs are those as described in handbooks such as Rote Liste 2014, for example, without limitation, main groups 12 (anti-diabetic drugs) or 86 (oncology drugs), and Merck Index, 15th edition.

Examples of APIs for the treatment and/or prophylaxis of type 1 or type 2 diabetes mellitus or complications associated with type 1 or type 2 diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the terms "analogue" and "derivative" refers to a polypeptide which has a molecular structure which formally can be derived from the structure of a naturally occurring peptide, for example that of human insulin, by deleting and/or exchanging at least one amino acid residue occurring in the naturally occurring peptide and/or by adding at least one amino acid residue. The added and/or exchanged amino acid residue can either be codeable amino acid residues or other naturally occurring residues or purely synthetic amino acid residues. Insulin analogues are also referred to as "insulin receptor ligands". In particular, the term "derivative" refers to a polypeptide which has a molecular structure which formally can be derived from the structure of a naturally occurring peptide, for example that of human insulin, in which one or more organic substituent (e.g. a fatty acid) is bound to one or more of the amino acids. Optionally, one or more amino acids occurring in the naturally occurring peptide may have been deleted and/or replaced by other amino acids, including non-codeable amino acids, or amino acids, including non-codeable, have been added to the naturally occurring peptide.

Examples of insulin analogues are Gly(A21), Arg(B31), Arg(B32) human insulin (insulin glargine); Lys(B3), Glu(B29) human insulin (insulin glulisine); Lys(B28), Pro(B29) human insulin (insulin lispro); Asp(B28) human insulin (insulin aspart); human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Examples of insulin derivatives are, for example, B29-N-myristoyl-des(B30) human insulin, Lys(B29) (N- tetradecanoyl)-des(B30) human insulin (insulin detemir, Levemir^{®}); B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin, B29-N-omega-carboxypentadecanoyl-gamma-L-glutamyl-des(B30) human insulin (insulin degludec, Tresiba^{®}); B29-N-(N-lithocholyl-gamma-glutamyl)-des(B30) human insulin; B29-N-(ω-carboxyheptadecanoyl)-des(B30) human insulin and B29-N-(w-carboxyheptadecanoyl) human insulin.

Examples of GLP-1, GLP-1 analogues and GLP-1 receptor agonists are, for example, Lixisenatide (Lyxumia^{®}), Exenatide (Exendin-4, Byetta^{®}, Bydureon^{®}, a 39 amino acid peptide which is produced by the salivary glands of the Gila monster), Liraglutide (Victoza^{®}), Semaglutide, Taspoglutide, Albiglutide (Syncria^{®}), Dulaglutide (Trulicity^{®}), rExendin-4, CJC-1134-PC, PB-1023, TTP-054, Langlenatide / HM-11260C (Efpeglenatide), HM-15211, CM-3, GLP-1 Eligen, ORMD-0901, NN-9423, NN-9709, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, ZP-DI-70, TT-401 (Pegapamodtide), BHM-034. MOD-6030, CAM-2036, DA-15864, ARI-2651, ARI-2255, Tirzepatide (LY3298176), Bamadutide (SAR425899), Exenatide-XTEN and Glucagon-Xten.

An example of an oligonucleotide is, for example: mipomersen sodium (Kynamro^{®}), a cholesterol-reducing antisense therapeutic for the treatment of familial hypercholesterolemia or RG012 for the treatment of Alport syndrome.

Examples of DPP4 inhibitors are Linagliptin, Vildagliptin, Sitagliptin, Denagliptin, Saxagliptin, Berberine.

Examples of hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Examples of polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 (Synvisc^{®}), a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region. The term antibody also includes an antigen-binding molecule based on tetravalent bispecific tandem immunoglobulins (TBTI) and/or a dual variable region antibody-like binding protein having cross-over binding region orientation (CODV).

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, tetraspecific and multispecific antibodies (e.g., diabodies, triabodies, tetrabodies), monovalent or multivalent antibody fragments such as bivalent, trivalent, tetravalent and multivalent antibodies, minibodies, chelating recombinant antibodies, tribodies or bibodies, intrabodies, nanobodies, small modular immunopharmaceuticals (SMIP), bindingdomain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Examples of antibodies are anti PCSK-9 mAb (e.g., Alirocumab), anti IL-6 mAb (e.g., Sarilumab), and anti IL-4 mAb (e.g., Dupilumab).

Pharmaceutically acceptable salts of any API described herein are also contemplated for use in a drug or medicament in a drug delivery device. Pharmaceutically acceptable salts are for example acid addition salts and basic salts.

Those of skill in the art will understand that modifications (additions and/or removals) of various components of the APIs, formulations, apparatuses, methods, systems and embodiments described herein may be made without departing from the full scope and spirit of the present invention, which encompass such modifications and any and all equivalents thereof.

An example drug delivery device may involve a needle-based injection system as described in Table 1 of section 5.2 of ISO 11608-1:2014(E). As described in ISO 11608-1:2014(E), needle-based injection systems may be broadly distinguished into multi-dose container systems and single-dose (with partial or full evacuation) container systems. The container may be a replaceable container or an integrated non-replaceable container.

As further described in ISO 11608-1:2014(E), a multi-dose container system may involve a needle-based injection device with a replaceable container. In such a system, each container holds multiple doses, the size of which may be fixed or variable (pre-set by the user). Another multi-dose container system may involve a needle-based injection device with an integrated non-replaceable container. In such a system, each container holds multiple doses, the size of which may be fixed or variable (pre-set by the user).

As further described in ISO 11608-1:2014(E), a single-dose container system may involve a needle-based injection device with a replaceable container. In one example for such a system, each container holds a single dose, whereby the entire deliverable volume is expelled (full evacuation). In a further example, each container holds a single dose, whereby a portion of the deliverable volume is expelled (partial evacuation). As also described in ISO 11608-1:2014(E), a single-dose container system may involve a needle-based injection device with an integrated non-replaceable container. In one example for such a system, each container holds a single dose, whereby the entire deliverable volume is expelled (full evacuation). In a further example, each container holds a single dose, whereby a portion of the deliverable volume is expelled (partial evacuation).

## Claims

1. A method of detecting a dose of a medicament administered using a drug delivery device, the drug delivery device comprising a dosage dial and a dialled dose indication notch, the dosage dial comprising a plurality of notches, the method comprising:
dialling a dose;
determining, after dialling a dose, a distance (L6) between the dosage dial (12) and a body (10) of the drug delivery device (1);
detecting the relative position of the dialled dose indication notch and the notches provided on the dosage dial;
determining the dialled dose based on the detected distance (L6) and the relative position of the dialled dose indication notch and the notches provided on the dosage dial.

2. The method of claim 1, wherein the distance (L6) between the dosage dial (12) and a body (10) of the drug delivery device (1) corresponds in a predefined manner to a dose displayed in a dosage window (13) of the drug delivery device.

3. The method of claim 2, further comprising:
recognizing, using OCR software provided in a user device (2, 3), the dose displayed in the dosage window (13); and
correlating the dose displayed in the dosage window (13) with the distance (L6) between the dosage dial (12) and a body (10) of the drug delivery device (1).

4. The method of any of claim 1 to 3, comprising:
determining that an even dose has been dialled when one of the notches provided on the dosage dial is aligned with the of the dialled dose indication notch or is within 0.5mm of the dialled dose indication notch; and
determining that an odd dose has been dialled when the dialled dose indication notch is positioned between two of the notches provided on the dosage dial.

5. A user device (2, 3) comprising a processor, a memory, a display and a camera, wherein the memory of the user device stores instructions which, when executed by the processor, cause the user device to:
capture, using the camera of the user device, at least one image and/or video of a drug delivery device, the drug delivery device comprising a dosage dial and a dialled dose indication notch, the dosage dial comprising a plurality of notches;
determine, using the captured at least one image and/or video of the drug delivery device and after dialling a dose into the drug delivery device, a distance (L6) between the dosage dial (12) and a body (10) of the drug delivery device (1);
detecting, using the captured at least one image and/or video of the drug delivery device, the relative position of the dialled dose indication notch and the notches provided on the dosage dial;
determining, using the captured at least one image and/or video of the drug delivery device, the dialled dose based on the detected distance (L6) and the relative position of the dialled dose indication notch and the notches provided on the dosage dial.

6. The user device (2, 3) of claim 5, wherein the distance (L6) between the dosage dial (12) and a body (10) of the drug delivery device (1) corresponds in a predefined manner to a dose displayed in a dosage window (13) of the drug delivery device.

7. The user device of claim 6, wherein the instructions further cause the user device to:
recognize, using OCR software provided in the user device (2, 3), the dose displayed in the dosage window (13); and
correlate the dose displayed in the dosage window (13) with the distance (L6) between the dosage dial (12) and a body (10) of the drug delivery device (1).

8. The user device (2, 3) of any of claim 5 to 7, wherein the instructions further cause the user device to:
determine that an even dose has been dialled when one of the notches provided on the dosage dial is aligned with the of the dialled dose indication notch or is within 0.5mm of the dialled dose indication notch; and
determine that an odd dose has been dialled when the dialled dose indication notch is positioned between two of the notches provided on the dosage dial.

9. A system comprising the user device (2, 3) of any of claim 5 to 8 and the drug delivery device (1), wherein the body (10) of the drug delivery device and/or the dosage dial (12) is provided with one or more contrast features.

10. The system of claim 9, wherein the contrast features comprise features which are provided in contrast colour and/or features protruding above the surrounding features.

11. The system of claim 9 or claim 10, wherein the contrast feature comprises a first collar (109a) provided on the dosage dial (12) and/or a second collar (109b) provided on the body (10) of the drug delivery device.

12. The system of claim 11, wherein the second collar (109b) is positioned on the end of the body (10) opposite to a needle (15) of the drug delivery device, such that the second collar (109b) faces the dosage dial (12).

13. The system of claim 11 or claim 12, wherein the first collar (109a) is provided in a colour different from the colour of the dosage dial (12) and the second collar (109b) is provided in a colour different from the colour of the body (10) of the drug delivery device (1).

14. The system of any of claims 11 to 13, wherein an outermost diameter of the first collar (109a) is larger than an outermost diameter of the body (10) of the drug delivery device (1), such that the first collar (109a) forms a ring-like structure protruding from the dosage dial (12).

15. The system of any of claims 11 to 14, wherein an outermost diameter of the second collar (109b) is larger than an outermost diameter of the body (10) of the drug delivery device (1), such that the second collar (109b) forms a ring-like structure protruding from the body (10).
